# EUROPEAN PATENT SPECIFICATION

(11) **EP 0 727 183 B1**
(45) Date of publication and mention of the grant of the patent: **27.11.2002**
(21) Application number: 96101807.4
(22) Date of filing: 08.02.1996
(51) Int. Cl.: A61B 5/042, A61B 18/12

(54) **A catheter, particularly for the treatment of cardiac arrhythmia**
Katheter, insbesondere zur Behandlung von Herzarrhythmie
Cathéter, notamment pour le traitement de l'arythmie cardiaque

(30) Priority: 14.02.1995 IT TO950099
(43) Date of publication of application: 21.08.1996
(73) Proprietor: SORIN BIOMEDICA CARDIO S.p.A., 13040 Saluggia (Vercelli) (IT)
(72) Inventor: Rolando, Giovanni, I-10034 Chivasso (Torino) (IT); Garberoglio, Bruno, I-10156 Torino (IT)
(74) Representative: Bosotti, Luciano

(56) References cited:
- EP-A- 0 500 215
- EP-A- 0 573 311
- US-A- 5 293 869
- US-A- 5 345 936

## Description

The present invention relates to catheters usable for the treatment of cardiac arrhythmia.

It is known that the heart muscle, the function of which is essentially to act as a pump to maintain the circulation of the blood in the body, may be subject to alterations in the contraction mechanism, generally known as arrhythmia, which may affect the various chambers of the heart. In particular, arrhythmia may affect the left ventricle, that is, the part of the heart the function of which is to force the blood into the circulatory system.

The contractions of the heart muscle upon which the operation of the heart is based result from the application of electrical waves. In deliberately simplified terms, these waves can be seen as being generated in the atrium and being propagated through the atrio-ventricular connection towards the ventricles, then proceeding in a centrifugal direction through the conductive tissue of the endocardium.

Essentially, the abnormality defined as arrhythmia can be attributed to a change in the propagation of the excitation wave through the heart muscle. In deliberately simplified terms (but according to concepts well known in the medical field) arrhythmia can be attributed essentially to local short-circuiting of the propagation of the excitation waves. These cause the muscle to be excited and to contract before this is actually required for the heart function to be performed correctly.

The regions of the heart wall in which arrhythmia phenomena are triggered (which are usually located in the endocardium but may also be situated in the thickness of the myocardium) are currently known as arrhythmogenesis sites or foci (or ectopic foci).

In addition to pharmacological treatment which has intrinsic disadvantages, a treatment for arrhythmia which is now very widespread provides for the location of the foci within the heart chamber concerned by an operation currently known as "mapping" and the subsequent application of localized energy (for example heat or electromagnetic energy) so as to bring about passivation of the arrhythmogenesis site by an operation currently known as "ablation".

In recent years, extensive research activities have been dedicated to solving the problem of rendering both the mapping operation and the ablation operation effective and reliable (particularly as regards speed of operation and the precision of the operation which is decisive in clearing up the arrhythmic syndrome). This particularly concerns the possibility of performing these operations by means of catheterization according to a solution which is now established as greatly preferable in order to avoid the need to perform open chest surgery.

This research activity is documented by a corresponding number of patent documents, amongst which may be cited, by way of example, the documents US-A-4 522 212, US-A-5 237 996, US-A-5 293 869, US-A-5 345 936, EP-A-0 573 311 and WO-A-94/12098.

These prior documents address in detail some specific problems such as the construction of the mapping and/or ablation electrodes, the structure of the respective electrical connections, even along the respective catheters, the criteria which may be adopted for processing the signals, the generation and the transfer of the ablation energy towards the application site, etc.

Problems of various types continue to arise, however, in the production of catheters of the type specified above.

In the first place, an accurate mapping operation, particularly as regards precision and resolution in the identification of the ectopic foci presupposes that the mapping electrodes can be brought as much as possible into contact with the wall of the heart chamber investigated. From this point of view there is a certain conflict between the need to give the element of the catheter which carries the electrodes a certain "body" so that it can be manoeuvred at its proximal end in order to bring about an effective movement of the electrodes, and the need to ensure that the structures which support the electrodes are extremely flexible so as to be able to follow the shape of the heart chamber wall which is very uneven.

In this connection, it should not be forgotten that the mapping and ablation operations are normally carried out whilst conditions of tachycardia (typical conditions in which arrhythmia occurs) are induced in the patient and hence with the heart muscle subjected to intense excitation.

As far as the ablation electrode is concerned, there is therefore a need to be able to arrange for it to be brought rapidly and precisely to the ectopic site on which the ablation is to be carried out.

Precisely for the reasons explained above (that is, the fact that the operation is carried out whilst tachycardia is induced in the patient) it is essential to ensure that the entire operation (mapping/ablation) can be carried out in a short time, nominally of the order of minutes and not, as frequently still occurs, several hours.

The present invention primarily addresses the problem of providing a catheter structure having mapping means which are improved both as regards the identification of the ectopic foci and, in a preferred embodiment of the invention, as regards the possibility of integrating the ablation function in the catheter so as to create a single system which can effectively perform both the operations considered.

According to the present invention, this object is achieved by virtue of a catheter having the specific characteristics recited in the following claims.

The invention will now be described, purely by way of non-limiting example, with reference to the appended drawings, in which:
Figure 1 shows, in general, the structure of a composite mapping and ablation catheter formed according to the invention, illustrated in the working position in a heart chamber,
Figure 2 shows in greater detail the structure of one of the elements of the catheter of Figure 1,
Figure 3 shows in detail the structure of the element illustrated in Figure 2, according to a possible variant,
Figure 4 is a section taken on the line IV-IV of Figure 3,
Figure 5 shows in detail a possible optional characteristic of a catheter according to the invention, and
Figures 6 and 7 show two steps of the use of a catheter of another type formed according to the invention.

As a basic premise, it should be remembered that the present description relates primarily to the support structure of the mapping and ablation electrodes of the catheter. For this reason, the specific constructional details of the individual mapping electrodes, the respective electrical and functional connections, as well as the structure and the connections of the ablation electrode present in some preferred embodiments of the invention are not described in detail herein, since they are not relevant per se for an understanding and the implementation of the invention. For a general description of these concepts, reference may usefully be made to the prior documents cited in the introductory portion of the present description.

In the appended drawings, a mapping/ablation catheter usable for the treatment of cardiac arrhythmia is generally indicated 1.

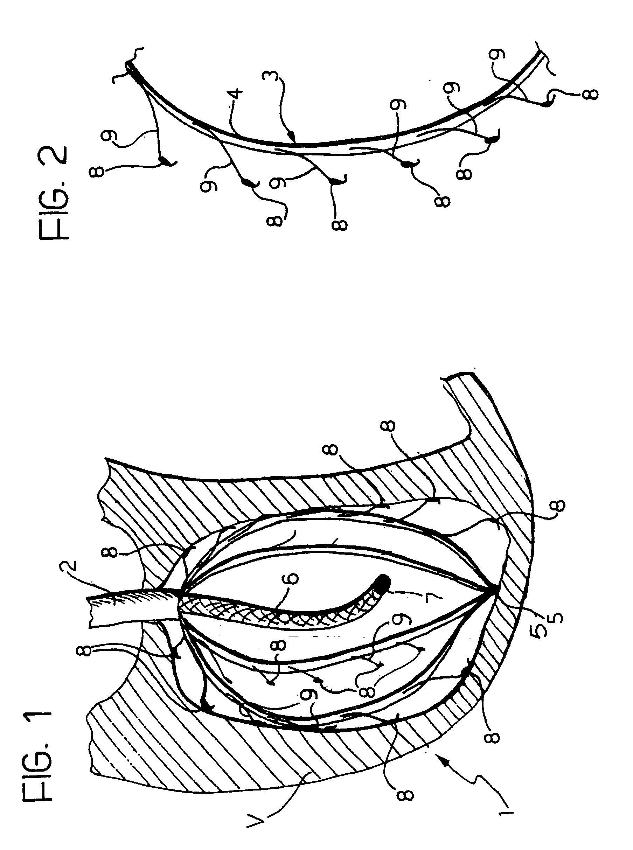

The catheter according to the invention is intended to be introduced into one of the chambers of the heart of a patient suffering from cardiac arrhythmia (for example, the left ventricle, indicated V in Figure 1). According to a known structure (see, for example, the document EP-A-0 573 311), the catheter 1 comprises a rod or shaft 2 having a proximal end (not visible in the drawings) which enables the operator to introduce the catheter into the patient's body percutaneously, causing it to go up through the circulatory system (for example, through the femoral artery), in use. This continues until the distal end of the catheter 1 is facing the inside of the chamber (the ventricle V in the example illustrated) inside which the ectopic foci at the root of the arrhythmic syndrome are to be located for subsequent ablation.

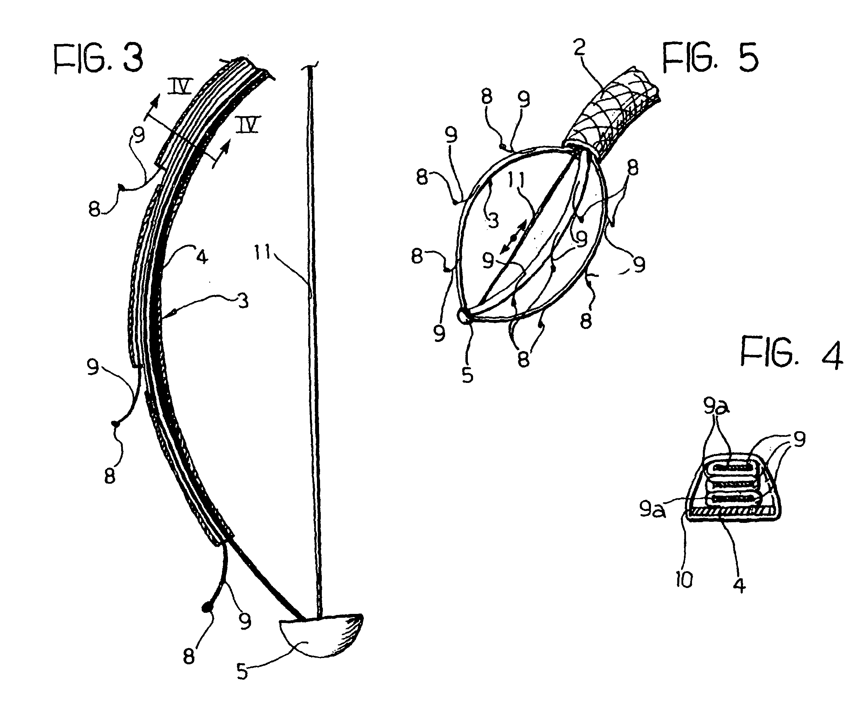

With reference again to the structure described in the document EP-A-0 573 311, the distal end of the catheter comprises a group of flexible arms 3 which branch out from the shaft 2 so as to form a kind of cage or basket comprising a set of flexible arcuate elements 4 (Figures 2 and 3) which are connected to one another at their distal ends at the point indicated 5 defining the top or tip of the cage or basket. It will be appreciated that, in a preferred embodiment, the basket in question has a general shape approximately comparable to that of an onion.

A central flexible element 6 carrying an ablation electrode 7 at its distal end can be introduced (in known manner) into the cage or basket in a central position from the shaft 2 of the catheter.

In the structure described, the structure of the shaft 2 of the catheter is generally assumed to be such that it comprises several elements (for example, sheaths) coaxial with one another, with the central ablation arm or catheter 7 extending in a central position with respective associated orientation members (not shown, but of known type) which enable the ablation catheter 7 to be brought selectively to the ectopic foci located by the mapping electrodes, generally indicated 8.

As can best be seen in the detailed view of Figure 2, which shows the structure of any one of the arms 3 of the cage or basket of the catheter shown in Figure 1, the mapping electrodes 8 are not located directly on the arcuate element 4 which defines a respective arm of the cage or basket but, on the contrary, on the ends of respective hair-like structures 9 formed so as to diverge sideways from the arcuate element 4 on the outer side thereof, that is, on the convex side (or rear surface) thereof which, in use, is intended to face the wall of the chamber V being mapped.

According to a solution which has been found preferable on the basis of the experiments carried by the Applicant up to now, it is possible to produce the arcuate support 4 with the use of a material having a high degree of flexibility (possibly in combination with shape memory characteristics) such as, for example, the material known as Nitinol. The use of equivalent materials is, however, of course possible.

In fact, it must be taken into account that, before being spread out inside the chamber V being mapped, the cage or basket situated at the distal end of the catheter 1 must be able to be gathered together inside the shaft 2 or, in any case, in a tubular sheath which surrounds the basket itself, and then to be uncovered by this containment member so that the arms 3 can spread out, consequently opening out the cage or basket inside the chamber V.

As regards the structure of the hair-like appendages 9, on the other hand, the selection currently considered preferable is that of a material such as that known as Kapton. This material combines various characteristics which are advantageous from a functional point of view: in the first place, it is a material which can be defined as a plastic material and hence a material which can be shaped and moulded with good electrical insulation properties and, in addition, with the possibility of conductive strips, for example, of metal being formed thereon by known techniques (for example, by thin-film technology or by evaporation) to enable the necessary connections to be made with the mapping electrodes 8.

The use of hair-like appendages, which are usually formed so as to be selectively softer (that is, more yielding) than the arcuate element 4 and any number of which may generally be provided for each arm 3 of the cage or basket of the catheter, is in fact advantageous, with regard to the possibility of locating the mapping electrodes 8 precisely in contact with the wall of the heart chamber V being mapped.

In this connection, it will be appreciated that - owing to its intrinsic nature - the arm 3 and, in particular, the resilient arcuate element 4 which acts as a support structure, tends to follow the shape of the internal wall of the heart chamber V in a complementary manner; however, for obvious physical reasons, precisely because of its overall length, the support structure necessarily tends to be brought to a tangential position relative to the wall (in other words, to be inscribed within the wall in question) so that it is impossible to bring its entire length into contact with the heart wall which would, however, be desirable in order to achieve the necessary contact of the electrodes. As already stated above, the internal wall of the heart cavity has a fairly uneven shape which is also due to the presence of the chordae tendineae which constitute further elements which may oppose the movement of the electrodes towards the chamber wall.

Precisely because of their flexibility, the hair-like structures 9 according to the invention, on the other hand, enable the electrodes 8 to penetrate and advance locally against the heart wall so as to bring the electrodes precisely to the desired position. The correct carrying-out of the mapping operation presupposes that the various electrodes 8 of the catheter are simultaneously in contact with the wall of the chamber V so as to permit the detection and processing of the electrical signals which come simultaneously from the various electrodes 8 of the catheter.

The solution of associating extendible structures carrying mapping electrodes at their ends with generally arcuate elements comparable to the elements 3 of the solution according to the invention was proposed by the document US-A-4 522 212 (see in particular Figure 13 and the description relating thereto). It will be noted, however, that, in this prior solution, the extendible elements constitute further branches or forks which extend from the distal ends of the aforesaid arcuate elements, acting as main branches of the catheter. The solution proposed by US-A-4 522 212 does not therefore solve the problem of preventing the incorrect positioning of the electrodes resulting from the generally tangential arrangement of the arcuate elements of the catheter relative to the endocardial wall. This problem is solved, however, by the solution according to the invention, in which the hair-like structures 9 constitute lateral branches and not end branches of the respective arcuate element.

Figure 3 shows, in greater detail, a possible variant in which the construction of the arcuate structure 3 provides for the basic element 4 formed of a material such as Nitinol to be given a generally flattened shape (see, for example, the sectioned view of Figure 4). This enables the hair-like structures 9 which carry the electrodes 8 to be produced in the form of further strips (see Figure 4 again) housed in respective guide sheaths 9a arranged one against another in a pack and disposed inside an outer sheath 10 formed, for example, of heat-shrinking material, so as to produce a compact structure.

Thus, as can be appreciated better from an observation of Figures 3 and 4 in combination, according to the variant, each of the hair-like structures 9 constitutes the distal end, which is resiliently prestressed so as to be able to project outwardly from the arcuate element 4, of a corresponding elongate structure which can extend inside the shaft 2 of the catheter and (according to a known solution) be connected to a respective manipulator member situated at the proximal end of the catheter which is located outside the patient's body. With the use of this solution, each hair-like end 9 can be manoeuvred by being made to project to a greater or lesser extent from the corresponding sheath 9a and from the sheath 10 (again, see Figure 3) so as to be able, for example, to favour the movement of the respective electrode 8 towards the endocardial wall.

Figures 3 and 5 show how the tip portion 5 of the cage or basket can be connected to a thread-shaped element 11 which is also intended to extend inside the shaft of the catheter 2 towards a corresponding manipulation member situated at the proximal end of the catheter. This enables the general shape assumed by the cage or basket to be varied as a result of an operation to adjust the axial position of the thread 11 relative to the shaft 2 of the catheter. In general, if the thread 11 is withdrawn within the shaft 2 of the catheter the cage or basket assumes a more bulbous or rounded shape. On the other hand, if the thread 11 is slackened, favouring the movement of the tip 5 away from the shaft 2 of the catheter, the cage or basket assumes a generally more slender shape.

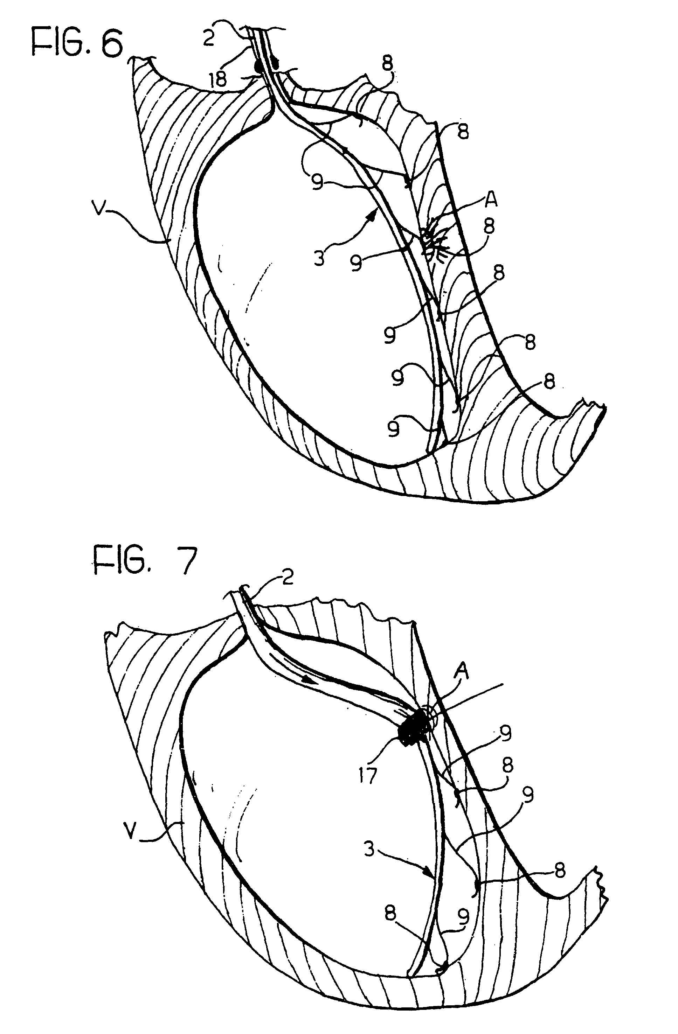

Figures 6 and 7 show how the same principle (the mounting of the mapping electrodes 8 at the ends of hair-like structures 9 projecting from a respective arcuate support 3 of the catheter) can advantageously be used to achieve precise positioning of an ablation electrode, indicated 17.

For simplicity of illustration, Figures 6 and 7 refer theoretically to a catheter comprising a single arcuate structure 3 possibly having a shaping thread such as the thread 11 (not shown specifically in the drawings). In any case, the same solution can also be applied to catheters in which there are several arcuate structures 3.

In this case, the ablation electrode 17 is situated at the distal end of a sheath 18 which can be slid inside or along the shaft of the catheter 2. For simplicity, an ablation electrode 17 having a generally annular shape is referred to in this case. This electrode can be located at the distal end of a sheath 18 formed so as to slide outside the shaft 2 of the catheter which carries the arcuate structure 3 on which the mapping electrodes 8 are mounted by means of the hair-like structures 9.

The solution described is ideally suitable for locating the ablation electrode 17 on or at least closely adjacent the mapping electrode 8 which supplies the signal identifying the existence of an arrhythmogenesis site.

A site of this type is indicated A in Figure 6, by way of example.

In this case, the corresponding electrode 8 (together with the other electrodes 8, in dependence on the processing of the mapping signals carried out by known methods) supplies the information which identifies the site A.

This indication which is presented externally to the operator, enables the operator to slide the sheath 18 so as to move the ablation electrode 17 along the arcuate structure 3 until the electrode 17 is brought to the site A identified by the corresponding electrode.

Precisely because of the intrinsically resilient nature of the hair-like structures 9, the movement of the sheath 18 is not appreciably obstructed. In fact, as it descends along the arcuate structures 3, the sheath 18 bends the hair-like structures 9 which it meets along its path, bringing the electrodes 8 against the arcuate structure, all with negligible resistance to the movement. Once the ablation electrode 17 is located at the site A, the electrode 17 can be activated (in known manner) so as to perform the desired ablation operation.

Upon completion of the operation, the catheter as a whole can be withdrawn, causing the arcuate structure or structures 3 to bend back and possibly collecting them inside the shaft 2 of the catheter. A similar operation can be carried out on the ablation electrode (7 or 17, in the two embodiments of Figures 1 and 7) and on the respective associated members so that the catheter as a whole can be brought back outside the patient's body.

## Claims

1. A catheter for the treatment of cardiac arrhythmia, comprising at least one arcuate structure (3) which, in use, is capable of extending inside a heart chamber (V) being mapped and which carries at least one associated mapping electrode (8), **characterized in that** the at least one electrode (8) is mounted on a hair-like structure (9) made of a relatively softer material than the arcuate structure (3), and **in that** the arcuate structure (3) has a respective, generally convex, outer side, or rear surface, and **in that** the hair-like structure (9) extends leterally so as to project from the outer side of the arcuate structure (3).

2. A catheter according to Claim 1, **characterized in that** the arcuate structure (3) comprises a core (4) of material with good resilience such as Nitinol.

3. A catheter according to Claim 1, **characterized in that** the hair-like structure (9) is made of Kapton.

4. A catheter according to any one of the preceding claims, **characterized in that** the hair-like structure (9) constitutes the distal end of a lamellar element substantially co-extensive with the catheter (2) and movable along the length of the catheter (2), the arrangement being such that the length of the hair-like structure (9) is selectively adjustable.

5. A catheter according to Claim 4, **characterized in that** the arcuate structure (3) has a generally sandwich-like structure with a first layer (4) forming a basic arcuate element for the arcuate structure and at least one further layer (9) the distal end of which forms the hair-like structure and carries a respective mapping electrode (8).

6. A catheter according to Claim 5, **characterized in that** the arcuate structure (3) is housed in a tubular sheath (10).

7. A catheter according to Claim 6, **characterized in that** the sheath is made of a heat-shrinkable material.

8. A catheter according to Claim 6 or Claim 7, **characterized in that** the at least one further layer (9) is housed in a respective guide sheath (9a).

9. A catheter according to any one of the preceding claims, **characterized in that** it also comprises an ablation electrode (17) which can slide on the arcuate structure (3), with respective associated movement means (18) for positioning the ablation electrode (17) on at least one mapping electrode (8) as a result of a sliding movement with bending of the hair-like structure or structures (9).

10. A catheter according to Claim 9, **characterized in that** the movement means (19) comprise a tubular sheath slidable along the arcuate structure (3).

11. A catheter according to any one of the preceding claims, **characterized in that** it comprises a plurality of said arcuate structures (3) together forming a cage or basket-like structure, the arcuate structures (3) being connected to one another in a tip region (5) of the cage or basket, and **in that** the catheter comprises a shaping thread (11) for the cage or basket, the thread (11) extending towards the tip portion (5) so that the longitudinal translation of the shaping thread brings about a change in the shape of the cage or basket.

## Patentansprüche

1. Ein Katheter zur Behandlung kardialer Arrhythmien, der wenigstens ein bogenförmiges Element (3) umfaßt, das sich während des Gebrauchs innerhalb einer abzubildenden Herzkammer (V) ausdehnen kann, und das wenigstens eine zugeordnete Abbildungselektrode (8) hält, **dadurch gekennzeichnet, daß**
die wenigstens eine Elektrode (8) an einem haarartigen Aufbau (9), der aus einem verhältnismäßig weicheren Material besteht als das bogenförmige Element (3), befestigt ist,
das gebogene Element (3) eine entsprechende, im wesentlichen konvexe Außenseite oder hintere Oberfläche aufweist, und
der haarartige Aufbau (9) sich seitlich erstreckt, so daß er von der Außenseite des bogenförmigen Elementes (3) vorsteht.

2. Katheter nach Anspruch 1, **dadurch gekennzeichnet, daß** das bogenförmige Element (3) einen Kern (4) aus einem Material mit guter elastischer Verformbarkeit wie beispielsweise Nitinol umfaßt.

3. Katheter nach Anspruch 1, **dadurch gekennzeichnet, daß** der haarartige Aufbau (9) Kapton umfaßt.

4. Katheter nach einem der vorausgehenden Ansprüche, **dadurch gekennzeichnet, daß** der haarartige Aufbau (9) das distale Ende eines lamellierten Elementes, das im wesentlichen gemeinsam mit dem Katheter (2) ausdehnbar und entlang der Länge des Katheters (2) bewegbar ist, umfaßt, wobei die Anordnung so geartet ist, daß die Länge des haarartigen Aufbaus (9) wahlweise einstellbar ist.

5. Katheter nach Anspruch 1, **dadurch gekennzeichnet, daß** das bogenförmige Element (3) einen im wesentlichen sandwich-artigen Aufbau mit einer ersten Schicht (4), die ein bogenförmiges Basiselement für das bogenförmige Element ausbildet, und wenigstens einer weiteren Schicht (9), deren distales Ende den haarartigen Aufbau ausbildet und eine entsprechende Abbildungselektrode (8) hält, aufweist.

6. Katheter nach Anspruch 5, **dadurch gekennzeichnet, daß** das bogenförmige Element (3) in einer rohrförmigen Hülse (10) angeordnet ist.

7. Katheter nach Anspruch 6, **dadurch gekennzeichnet, daß** die Hülse aus einem wärmeschrumpfbaren Material hergestellt ist.

8. Katheter nach Anspruch 6 oder 7, **dadurch gekennzeichnet, daß** die wenigstens eine weitere Schicht (9) in einer entsprechenden Führungshülse (9a) angeordnet ist.

9. Katheter nach einem der vorausgehenden Ansprüche, **dadurch gekennzeichnet, daß** der Katheter weiterhin eine Ablationselektrode (17), die auf dem bogenförmigen Element (3) gleiten kann, mit einem entsprechenden Bewegungsmittel (18) zum Positionieren der Ablationselektrode (17) an wenigstens einer Abbildungselektrode (8) infolge einer Gleitbewegung mit Biegen des haarartigen Aufbaus oder der haarartigen Aufbauten, umfaßt.

10. Katheter nach Anspruch 9, **dadurch gekennzeichnet, daß** die Bewegungsmittel (18) eine rohrförmige Hülse aufweisen, die entlang des bogenförmigen Elementes (3) verschiebbar ist.

11. Katheter nach einem der vorausgehenden Ansprüche, **dadurch gekennzeichnet, daß** der Katheter weiterhin eine Vielzahl von genannten bogenförmigen Elementen (3) umfaßt, die gemeinsam eine käfig- oder korbartige Form ausbilden, wobei die bogenförmigen Elemente (3) an einem Spitzenbereich (5) des Käfigs oder Korbes miteinander verbunden sind, und daß der Katheter ein drahtartiges Formelement (11) für den Käfig oder Korb aufweist, wobei sich das drahtartige Formelement (11) in Richtung des Spitzenbereiches (5) erstreckt, so daß die longitudinale Verschiebung des drahtartigen Formelementes zu einer Formänderung des Käfigs oder Korbes führt.

## Revendications

1. Cathéter pour le traitement de l'arythmie cardiaque, comprenant au moins une structure arquée (3) qui, lors de son utilisation, peut s'étendre jusque dans une cavité du coeur (V) que l'on cartographie, et qui comprend au moins une électrode de cartographie (8) connexe, **caractérisé en ce que** ladite électrode (8) est montée sur une structure semblable à des poils (9) faite d'un matériau relativement plus mou que la structure arquée (3), **en ce que** ladite structure arquée (3) possède un côté externe essentiellement convexe respectif, ou surface arrière, et **en ce que** ladite structure semblable à des poils (9) s'étend latéralement de manière à dépasser dudit côté externe de la structure arquée (3).

2. Cathéter, selon la revendication 1, **caractérisé en ce que** la structure arquée (3) comprend une âme (4) faite d'un matériau ayant une bonne résilience, tel que du Nitinol.

3. Cathéter, selon la revendication 1, **caractérisé en ce que** la structure semblable à des poils (9) est faite de Kapton.

4. Cathéter, selon l'une quelconque des revendications précédentes, **caractérisé en ce que** la structure semblable à des poils (9) représente l'extrémité distale d'un élément lamellaire s'étendant essentiellement dans la même direction que le cathéter (2) et pouvant se déplacer sur la longueur dudit cathéter (2), la disposition étant telle que la longueur de la structure semblable à des poils (9) peut être ajustée de façon sélective.

5. Cathéter, selon la revendication 4, **caractérisé en ce que** la structure arquée (3) consiste essentiellement en une structure de type sandwich, laquelle comprend une première couche (4) formant un élément de base arqué de la structure arquée, et au moins une couche supplémentaire (9) dont l'extrémité distale constitue la structure semblable à des poils et supporte une électrode de cartographie respective (8).

6. Cathéter, selon la revendication 5, **caractérisé en ce que** la structure arquée (3) est abritée dans un fourreau tubulaire (10).

7. Cathéter, selon la revendication 6, **caractérisé en ce que** le fourreau est fait d'un matériau thermorétractable.

8. Cathéter, selon la revendication 6 ou la revendication 7, caractérisé en ce ladite et au moins une couche supplémentaire (9) est abritée dans un fourreau de guidage respectif (9a).

9. Cathéter, selon l'une quelconque des revendications précédentes, **caractérisé en ce qu'**il comprend également une électrode d'ablation (17) pouvant coulisser sur la structure arquée (3), ainsi qu'un moyen de déplacement connexe respectif (18) permettant de placer l'électrode d'ablation (17) sur au moins une électrode de cartographie (8) à la suite d'un mouvement coulissant et de la courbure de la ou des structures semblables à des poils (9).

10. Cathéter, selon la revendication 9, **caractérisé en ce que** le moyen de déplacement (19) comprend un fourreau tubulaire pouvant coulisser le long de la structure arquée (3).

11. Cathéter, selon l'une quelconque des revendications précédentes, **caractérisé en ce qu'**il comprend plusieurs desdites structures arquées (3) qui forment ensemble une structure de type cage ou panier, lesdites structures arquées (3) étant reliées les unes aux autres au niveau d'une zone d'extrémité (5) de la cage ou du panier, et **en ce que** ledit cathéter comprend un filin (11) de mise en forme de la cage ou du panier, lequel filin (11) s'étend vers la zone d'extrémité (5) de sorte qu'un mouvement en translation longitudinal du filin de mise en forme se traduise par une modification de la forme de la cage ou du panier.
